# EUROPEAN PATENT SPECIFICATION

(11) **EP 1 325 754 B1**
(45) Date of publication and mention of the grant of the patent: **16.05.2007**
(21) Application number: 03075991.4
(22) Date of filing: 27.06.1997
(51) Int. Cl.: A61L 15/28, A61L 15/64

(54) **Wound dressing materials comprising collagen and oxidized cellulose**
Wundverbandmaterialien mit Kollagen und oxidierter Cellulose
Pansements pour blessures à base de collagène et de cellulose oxydée

(30) Priority: 28.06.1996 GB 9613682
(43) Date of publication of application: 09.07.2003
(62) Divisional of application: 97928372.8
(73) Proprietor: Johnson & Johnson Medical Ltd., Edinburgh EH2 4NH (GB)
(72) Inventor: Watt, Paul William, Steeton, West Yorkshire, BD20 6UN (GB); Harvey, Wilson, Carlton, North Yorkshire, BD23 3DW (GB); Wiseman, David, Dallas, Texas 75248 (US); Light, Nicholas, Gargrave, North Yorkshire, BD23 3RX (GB); Saferstein, Lowell, West Orange, New Jersey 07042 (GB); Cini, John, Beacon Falls, Connecticut 06403 (US)
(74) Representative: James, Anthony Christopher W.P.

(56) References cited:
- EP-A- 0 049 469
- EP-A- 0 140 596
- EP-A- 0 177 064
- EP-A- 0 562 862
- DATABASE WPI Section Ch, Week 8948 Derwent Publications Ltd., London, GB; Class A96, AN 89-348621 XP002045447 & CS 8 704 580 A (MARYSKA S), 13 October 1989 (1989-10-13)

## Description

The present invention relates to wound dressings comprising oxidized cellulose complexed to collagen that are suitable for chronic wound healing.

Oxidized cellulose is produced by the oxidation of cellulose, for example with dinitrogen tetroxide. This process converts primary alcohol groups on the saccharide residues to carboxylic acid group, forming uronic acid residues within the cellulose chain..The oxidation does not proceed with complete selectivity, and as a result hydroxyl groups on carbons 2 and 3 are occasionally converted to the keto form. These ketone units introduce an alkali labile link, which at pH 7 or higher initiates the decomposition of the polymer via formation of a lactone and sugar ring cleavage. As a result, oxidized cellulose is biodegradable and bioabsorbable under physiological conditions.

The preferred oxidized cellulose for practical applications is oxidized regenerated cellulose (ORC) prepared by oxidation of a regenerated cellulose, such as rayon. It has been known for some time that ORC has haemostatic properties. ORC has been available as a haemostatic product called SURGICEL (Registered Trade Mark of Johnson & Johnson Medical, Inc.) since 1950. This product is produced by the oxidation of a knitted rayon material.

A modification of porosity, density and knit pattern led to the launch of a second ORC fabric product, INTERCEED (Registered Trade Mark - Johnson & Johnson Medical, Inc.) which was shown to reduce the extend of post-surgical adhesions in abdominal surgery.

US-A-2517772 (Doub et al. ) describes improved haemostatic materials obtained by impregnating ORC fabric with thrombin.

EP-A-0437095 describes a neutralised ORC material prepared by contacting an as-synthesised acidic ORC material with a solution of a basic salt of a weak organic acid, such as sodium acetate. The resulting neutralised product is indicated for haemostasis and adhesion prevention.

Collagen, which is a structural protein of animal origin, is known in various forms for use as a wound dressing material.

GB-A-1515963 describes cross-linked collagen-mucopolysaccharide composite materials for use in medical and surgical applications, blood vessel grafts and all forms of surgical prostheses. The composite material contains at least 0.5% by weight of a mucopolysaccharide irreversibly bound to collagen. The mucopolysaccharide is an animal polysaccharide containing hexosamine residues, such as hyaluronic acid, chondroitin sulphate or heparin sulphate. The composite materials are said to exhibit greater resistance to resorption and better blood compatibility than simple collagen materials.

US-A-4614794 and EP-A-0140596 describe complexes formed between collagen and polyanionic plant polysaccharides, such as sodium alginate. The complexes are preferably formed by combining the protein and the polysaccharide at a pH which is no higher than the isoelectric point of the protein. The resulting complexes are said to be suitable for a wide variety of medical and surgical applications, including wound dressings. There is no disclosure of the use of oxidized cellulose instead of the polyanionic plant polysaccharide. The specification also teaches that proteins other than collagen, such as fibrin or elastin may be used in the formation of useful protein/polysaccharide complexes.

GB-A-2280850 describes medicated implants for the treatment of periodontal disease comprising a medicated collagen film reinforced with a layer of bioresorbable polymer, which may be ORC. The collagen matrix may contain dispersed ORC fibers or fragments.

EP-A-0177064 describes the use of a knitted oxidised cellulose fabric as a surgical hemostat.

EP-A-0562862 describes bioabsorbable sponge materials for use as wound implants. The materials comprise a collagen sponge matrix having an oriented substructure therein. The matrix and/or substructures may comprise oxidised regenerated cellulose. There is no disclosure of the use of such materials for the treatment of chronic wounds.

GB-A-1006606 describes haemostatic wound dressings comprising oxidised cellulose crystallite aggregates dispersed in a gel matrix. The use of such haemostatic gels in surgery is disclosed.

CS-A-8704580 describes a textile dressing having a coating comprising collagen and ORC applied by dipping or spraying.

EP-A-0049469 describes freeze-dried haemostatic wound dressings based on a mixture of pepsin-solubilized collagen with a resorbable polymer selected from the group consisting of fibrinogen, SH-modified gelatine, SH-modified collagen, or SH-modified oxized cellulose.

The above-described collagen or collagen/polysaccharide wound dressing materials provide important advantages. The materials are of natural, biological origin (albeit sometimes chemically modified), and consequently tend to have low antigenicity. The materials are generally bioabsorbable, which reduces the trauma associated with removal of conventional wound dressing materials from the surface of the wound. Furthermore, some of these materials can have positive therapeutic effects on wound healing. For example, some animal mucopolysaccharides such as hyaluronic acid are thought to exert a chemotactic effect on wound healing cells such as fibroblasts, and thereby promote the growth and development of such cells. Nevertheless, there remains a need for improved wound dressing materials of this general type exhibiting still better control of physical properties and biological absorption rates, still better therapeutic effects on wound healing, and reduced cost.

It is an object of the present invention to provide improved wound dressing materials for mammalian, and especially human, chronic wounds, such as venous ulcers,decubitis ulcers and diabetic ulcers. Such chronic wounds generally exhibit little or no bleeding or adhesion to other body tissues, and accordingly oxidized cellulose would not previously have been indicated for the treatment of such wounds.

In a first aspect, the present invention provides a wound dressing material in the form of a freeze-dried or solvent-dried sponge, wherein said material consists of:
fibrous, substantially insoluble collagen;
oxidized cellulose in the form of milled fibers or powder, said oxidized cellulose being complexed to the collagen;
0 to 25% of biocompatible polysaccharides other than oxidized cellulose
0 to 20% of water and
0 to 40% of a plasticizer by weight.

Preferably, the oxidized cellulose is oxidized regenerated cellulose (ORC). Preferably, the oxidised cellulose has an average molecular weight greater than 50,000. Such oxidised cellulose is substantially insoluble in wound fluids, but will undergo very gradual breakdown into bioresorbable fragments at physiological pH.

Preferably, the oxidized cellulose is not neutralized. However, the present invention encompasses the use of partially or completely neutralised materials as described in EP-A-0437095 for the preparation of medicaments for the treatment of chronic wounds as hereinbefore defined.

Preferably, the collagen (where present) and oxidized cellulose together make up at least 75% by weight of the wound dressing material, more preferably at least 90% by weight of the material. Other components of the material may include 0-25% by weight of one or more other biocompatible polysaccharides, for example alginates such as sodium alginate or calcium alginate, starch derivatives such as sodium starch glycolate, cellulose derivatives such as methyl cellulose or carboxymethyl cellulose, or glycosaminoglycans such as hyaluronic acid or its salts, chondroitin sulfate or heparan sulfate. The material may also comprise up to 20% by weight, preferably up to 10% by weight of water. The material may also contain 0-40% by weight, preferably 0-25% by weight of a plasticiser, preferably a polyhydric alcohol such as glycerol.

Preferably, the weight ratio of the collagen to oxidized cellulose is from 1:99 to 99.99:1. More preferably, the weight ratio is in the range 1:10 to 99.9:1, still more preferably it is in the range 2:1 to 95:1.

The wound dressing is a freeze-dried or solvent-dried bioabsorbable sponge for application to a chronic wound, the sponge suitably comprising from 0.1% to 50% w/w of oxidized cellulose and from 50% to 99.9% w/w of the collagen. Preferably, the average pore size of the sponge is in the region of 10-500µm, more preferably about 100-300µm.

The collagen may be collagen obtained from any natural source, including microbiological sources, but is preferably collagen obtained from bovine corium that has been rendered largely free of non-collagenous components, for example fat, non-collagenous proteins, polysaccharides and other carbohydrates as described in US Patents Nos. 4614794 and 4320201. The collagen may be Type I, II or III collagen, or may also be chemically modified collagen, for example an atelocollagen obtained by removing the immunogenic telopeptides from natural collagen.

Preferably, the oxidized cellulose comprises oxidized regenerated cellulose (ORC). The oxidized regenerated cellulose (ORC) can be obtained by the process described in US Patent No. 3122479. This material offers numerous advantages including the features that it is biocompatible, biodegradable, non-immunogenic and readily commercially available. ORC is available with varying degrees of oxidation and hence rates of degradation.

The ready availability of both collagen and ORC having a range of controllable properties means that the properties of the materials used in the present invention can be controlled to an exceptional degree. In particular, the rate of biological absorption, porosity and density of the materials can be controlled.

It has also been found, surprisingly, that the collagen/oxidized cellulose complexes used in the preferred aspect of the present invention have an excellent ability to bind to growth factors, in particular, platelet derived growth factor (PDGF).

The dressings according to the present invention may be used in a method of treatment of a chronic wound in a mammal, such as a decubitis ulcer, a venous ulcer or a diabetic ulcer. Preferably, the dressing is applied to the chronic wound for a period of at least 1 hour, more preferably at least 6 hours, and most preferably at least 12 hours. The treatment may be extended for several days or weeks, with dressing changes as appropriate, if necessary, for chronic wounds.

Without wishing to the bound by any theory, it is thought that the dressings according to the present invention promote chronic wound healing in at least some of the following ways. Firstly, the oxidized cellulose binds to growth factors such as PDGF, EGF and FGF to retain these growth factors at the wound site otherwise, such growth factors tend to be carried away from the wound site along with the wound exudate. The gradual breakdown of oxidized cellulose at physiological pH results in gradual release of the growth factors back into the wound. A second reason is that oxidized cellulose is fully bioresorbable and physiologically acceptable. A third reason may be that the oligosaccharide fragments produced by the breakdown of oxidized cellulose in vivo themselves promote chronic wound healing.

Preferably, the chronic wound is selected from the group consisting of venous ulcers, decubitis ulcers and diabetic ulcers. Preferably, the chronic wound is substantially or completely non-bleeding. The term "chronic wound" does not encompass a periodontal disorder or disease.

In a further aspect, the present invention provides a method of making a wound dressing material according to the present invention comprising the steps of: providing an aqueous dispersion of fibrous, substantially insoluble collagen; dispersing milled fibers or powder of oxidized cellulose in the aqueous dispersion with mixing; followed by removing water from the aqueous dispersion by freeze drying or solvent drying.

The optional, additional components in the materials according to the present invention are preferably included in the aqueous dispersion prior to removal of water from the aqueous dispersion.

Preferably, the pH of the dispersion is adjusted to pH 3-4.5. This pH range is less than the isoelectric pH of collagen.

Preferably, the aqueous dispersion contains 5-30mg/ml of collagen. Preferably, a collagen-based sponge is formed by lyophilisation substantially as described in US-A-2157224.

Preferably, the process further comprises treating the collagen and oxidised cellulose in the dispersion, or in the dried material, with a cross-linking agent such as carbodiimide, hexamethylene diisocyanate (HMDI) or glutaraldehyde.

Alternatively, cross-linking may be carried out dehydrothermally. The method of cross-linking can markedly affect the final product. For example, HMDI cross-links the primary amino groups on the protein within the complex, whereas carbodiimide cross-links carbohydrate on the ORC to primary amino groups on the protein.

The oxidized cellulose may be added to the aqueous dispersion of collagen in the form of a suspension of the oxidized cellulose, preferably at a comparable pH to the collagen suspension, following by mixing by stirring or homogenisation. Alternatively, dry fibers of oxidized cellulose may be immersed in the aqueous dispersion of collagen.

Specific embodiments of the present invention will now be described further, by way of example, with reference to the accompanying drawings, in which:

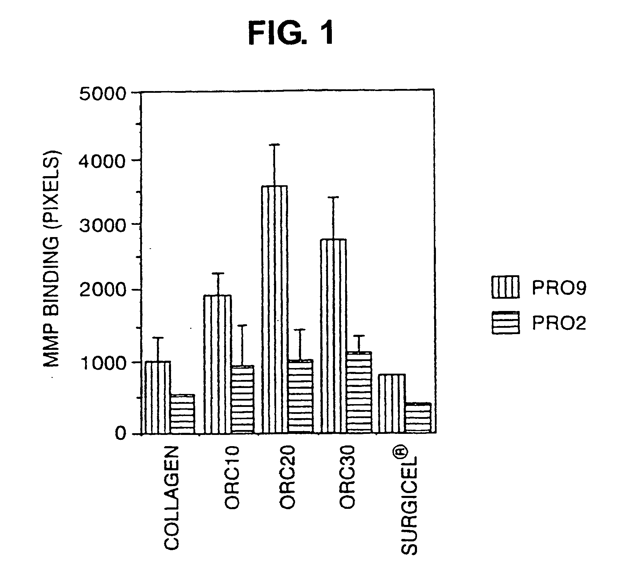

Figure 1 shows a graph of relative amounts of MMP binding measured for a collagen sponge and SURGICEL (RTM) ORC fabric (comparative measurements) and for sponges of collagen complexed with 10 wt%, 20wt% and 30 wt.% of fibrous ORC.

### Example 1: Preparation of a collagen/fibrous ORC sponge

Lyophilised collagen, prepared as described in US Patent No. 4614794 or 4320201, is resuspended in cold 0.05M acetic acid at a concentration of 10mg/ml. Milled ORC powder (milled Surgicel® cloth) is added to the suspension in a ratio of 1:3 ORC:collagen and homogenised using a Waring Blendor on low speed for 3 x 30s. The complex suspension is degassed in a vacuum oven for 10 min. and then poured to a depth of 3mm and blast frozen. The frozen suspension is then either freeze-dried and dehydrothermally cross-lined using a programmable Edwards freeze-drier with a temperature ramping facility, or dried using a solvent drying process as described in US-A-2157524.

### Procedure: Comparison of Matrix Metalloproteinase Binding

The effect of complexation between collagen and ORC on matrix metalloproteinase (MMP) binding was assessed as follows.

Collagen/fibrous ORC sponges containing 0%, 20% and 30% by weight of fibrous ORC were prepared by the procedure described in Example 1. An ORC-free collagen sponge was prepared for comparison purposes. A sample of SURGICEL (RTM) ORC fabric was also prepared for comparison.

Briefly 50mg of each material was placed in a 15ml plastic beaker containing 2.5ml of an acute wound fluid diluted to 1:50 in a proteolysis buffer (50mM tris/HCl pH7.8, 50mM CaCl., 0. 5M NaCl) and incubated at 37°C on a shaking water bath for 3 hours. Acute wound fluid contains various proteinases, including matrix metalloproteinases and many of these enzymes will preferentially bind to various dressing materials. The excess fluid absorbed by each material was mechanically expressed using a metal spatula and discarded. The remaining dressings were placed into pre-packed 2ml syringes (each syringe contained 0.5ml volume of 2.5mm glass beads). 4ml of proteolysis buffer was forced through the syringe in 1ml aliquots which were discarded. At this washing stage all of the unbound proteinases and proteinases which were only weakly bound to the dressing material had been removed from the dressing leaving the more tightly bound forms. The buffer rinsed dressings were then removed to another 15ml plastic beaker. 1ml of non-denaturing sample buffer (6.3ml 0.05M tris/HCl.c pH6.8, 2.5ml glycerol, 0.5g SDS, 16.2ml water and bromophenol blue) was added to each sample which were placed on an orbital shaker at setting six for 2 hours. The sample buffer detaches the tightly bound proteinases from the materials which are then present in the sample buffer itself. After this time 20 microliters of sample buffer was taken from each container and subjected to gelatin substrate SDS-polyacrylamide gel electrophoresis (zymography), as described by Heussen C. and Dowdle E.B., Anal., Biochem. 102:196-202 (1980).

The areas of the individual zones of clearance on the gels, which are due to proteinase activity, were accurately measured by the Optilab® system. This was achieved by repeating each binding experiment (n=3) and analyzing the results statistically by the Students T test, where P < 0.05. Analysis was against controls of pure collagen.

The results, shown in Figure 1, demonstrate a surprising synergistic improvement in MMP binding for the complexes of collagen with ORC. Data are presented for the proenzyme forms (PR02 and PR09) of matrix metalloproteinase 2 (Gelatinase A) and matrix metalloproteinase 9 (Gelatinase B).

Without wishing to be bound by any theory, it is thought that the improvement may be related to neutralization of opposing electrostatic charges on the collagen and the ORC by complexation.

## Claims

1. A wound dressing material in the form of a freeze-dried or solvent-dried sponge, wherein said material consists of :
fibrous, substantially insoluble collagen;
oxidized cellulose in the form of milled fibers or powder, said oxidized cellulose being complexed to the collagen;
0 to 25% of biocompatible polysaccharides other than oxidized cellulose
0 to 20% of water and
0 to 40% of a plasticizer by weight.

2. A wound dressing material according to claim 1, wherein the collagen and the oxidized cellulose together make up at least 75% by weight of the wound dressing material

3. A wound dressing material according to claim 1, comprising from 0.1 % to 50% by weight of the oxidized cellulose and from 50% to 99.9% by weight of the collagen.

4. A wound dressing material according to claim 1, consisting of 50% by weight of oxidized cellulose and 50% by weight of the collagen.

5. A wound dressing material according to claim 1, wherein the weight ratio of collagen to oxidized cellulose is from 2:1 to 95:1.

6. A wound dressing material according to any preceding claim that does not contain any biocompatible polysaccharides other than oxidized cellulose.

7. A wound dressing material according to any preceding claim that does not contain any plasticizer.

8. A wound dressing material according to any preceding claim, wherein the oxidized cellulose is oxidized regenerated cellulose.

9. A wound dressing material according to any preceding claim, wherein the material consists of collagen, oxidized regenerated cellulose and water.

10. A method of making a wound dressing material according to any of claims 1 to 9, comprising the steps of :
providing an aqueous dispersion of fibrous, substantially insoluble collagen;
dispersing milled fibers or powder of oxidized cellulose in the aqueous dispersion with mixing;
followed by removing water from the aqueous dispersion by freeze drying or solvent drying.

## Patentansprüche

1. Wundverbandmaterial in Form eines gefriergetrockneten oder lösungsmittelgetrockneten Schwammes, wobei das Material besteht aus:
faserigem, im Wesentlichen unlöslichem Kollagen;
oxidierter Zellulose in Form von Kurzfasern oder Puder, wobei die oxidierte Zellulose an das Kollagen komplexiert ist;
0 bis 25 % anderen biologisch verträglichen Polysacchariden als oxidierte Zellulose
0 bis 20 % Wasser und
0 bis 40 Gewichtsprozent eines Weichmachers.

2. Wundverbandmaterial nach Anspruch 1, wobei das Kollagen und die oxidierte Zellulose zusammen mindestens 75 Gewichtsprozent des Wundverbandmaterials ausmachen.

3. Wundverbandmaterial nach Anspruch 1, das von 0,1 bis 50 Gewichtsprozent oxidierte Zellulose und von 50 bis 99,9 Gewichtsprozent Kollagen umfasst.

4. Wundverbandmaterial nach Anspruch 1, das aus 50 Gewichtsprozent oxidierter Zellulose und 50 Gewichtsprozent Kollagen besteht.

5. Wundverbandmaterial nach Anspruch 1, wobei das Gewichtsverhältnis von Kollagen zu oxidierter Zellulose von 2:1 bis 95:1 beträgt.

6. Wundverbandmaterial nach einem der vorhergehenden Ansprüche, das keine anderen biologisch verträglichen Polysaccharide als oxidierte Zellulose enthält.

7. Wundverbandmaterial nach einem der vorhergehenden Ansprüche, das keinen Weichmacher enthält.

8. Wundverbandmaterial nach einem der vorhergehenden Ansprüche, wobei die oxidierte Zellulose oxidierte regenerierte Zellulose ist.

9. Wundverbandmaterial nach einem der vorhergehenden Ansprüche, wobei das Material aus Kollagen, oxidierter regenerierter Zellulose und Wasser besteht.

10. Verfahren zur Herstellung eines Wundverbandmaterials nach einem der Ansprüche 1 bis 9, das folgende Schritte umfasst:
Bereitstellen einer wässrigen Dispersion von faserigem, im Wesentlichen unlöslichem Kollagen;
Dispergieren von Kurzfasern oder Puder oxidierter Zellulose in der wässrigen
Dispersion durch Mischen;
gefolgt von
Entfernen des Wassers aus der wässrigen Dispersion durch Gefriertrocknung oder durch Lösungsmitteltrocknung.

## Revendications

1. Matériau de pansement sous la forme d'une éponge lyophilisée ou séchée par évaporation de solvant, dans lequel ledit matériau consiste en :
collagène fibreux substantiellement insoluble ;
cellulose oxydée sous la forme de fibres ou de poudre broyées, ladite cellulose oxydée formant un complexe avec le collagène ;
0 à 25 % de polysaccharides biocompatibles autres que de la cellulose oxydée,
0 à 20 % d'eau et
0 à 40 % d'un plastifiant en poids.

2. Matériau de pansement selon la revendication 1, dans lequel le collagène et la cellulose oxydée forment ensemble au moins 75 % en poids du matériau de pansement.

3. Matériau de pansement selon la revendication 1, comprenant entre 0,1 % et 50 % en poids de cellulose oxydée et entre 50 % et 99,9 % en poids de collagène.

4. Matériau de pansement selon la revendication 1, consistant en 50 % en poids de cellulose oxydée et 50 % en poids de collagène.

5. Matériau de pansement selon la revendication 1, dans lequel le rapport en poids du collagène par rapport à la cellulose oxydée est compris entre 2/1 et 95/1.

6. Matériau de pansement selon l'une quelconque des revendications précédentes, qui ne contient aucun polysaccharide biocompatible autre que la cellulose oxydée.

7. Matériau de pansement selon l'une quelconque des revendications précédentes, qui ne contient aucun plastifiant.

8. Matériau de pansement selon l'une quelconque des revendications précédentes, dans lequel la cellulose oxydée est de la cellulose oxydée régénérée.

9. Matériau de pansement selon l'une quelconque des revendications précédentes, dans lequel le matériau consiste en collagène, cellulose régénérée oxydée et eau.

10. Procédé de fabrication d'un matériau de pansement selon l'une quelconque des revendications 1 à 9, comprenant les étapes consistant à :
fournir une dispersion aqueuse de collagène fibreux substantiellement insoluble ;
disperser des fibres ou de la poudre broyées de cellulose oxydée dans la dispersion aqueuse en les mélangeant ;
suivies de l'étape consistant à retirer l'eau de la dispersion aqueuse par lyophilisation ou séchage du solvant.
